# FASCICULE DE BREVET EUROPEEN

(11) **EP 3 408 669 B1**
(45) Date de publication et mention de la délivrance du brevet: **23.02.2022**
(21) Numéro de dépôt: 16809836.6
(22) Date de dépôt: 15.12.2016
(51) Int. Cl.: G01N 33/24, G01N 15/08, G01N 23/04

(54) **SYSTEME ET PROCEDE DE MESURE D'UNE PROPRIETE D'ECOULEMENT D'UN FLUIDE AU SEIN D'UN MILIEU POREUX**
SYSTEM UND VERFAHREN ZUR MESSUNG EINER FLUSSEIGENSCHAFT EINER FLÜSSIGKEIT IN EINEM PORÖSEN MEDIUM
SYSTEM AND METHOD FOR MEASURING A FLOW PROPERTY OF A FLUID IN A POROUS MEDIUM

(30) Priorité: 29.01.2016 FR 1650711
(43) Date de publication de la demande: 05.12.2018
(73) Titulaire: IFP Energies nouvelles, 92500 Rueil-Malmaison (FR)
(72) Inventeur: YOUSSEF, Souhail, 93100 Montreuil (FR); PEYSSON, Yannick, 92500 Rueil Malmaison (FR); DESCHAMPS, Hervé, 78590 Noisy le Roi (FR)
(74) Mandataire: IFP Energies nouvelles
(86) Numéro de dépôt international: PCT/EP2016/081139
(87) Numéro de publication internationale: WO 2017/129312

(56) Documents cités:
- WO-A1-2008/132132
- WO-A1-2012/164090
- WO-A1-2016/087890
- US-A- 5 109 398
- US-A- 5 164 672
- REZKI OUGHANEM ET AL: "PORE-SCALE TO CORE-SCALE STUDY OF CAPILLARY DESATURATION CURVES USING MULTI-SCALE 3D IMAGING", IOR 2013 - FROM FUNDAMENTAL SCIENCE TO DEPLOYMENT, 19 septembre 2013 (2013-09-19), pages 16-19, XP055150813, DOI: 10.3997/2214-4609.20142615

## Description

La présente invention concerne le domaine des mesures de propriétés d'écoulement d'un fluide dans un milieu poreux, notamment un milieu poreux issu d'une formation souterraine. Les mesures peuvent servir notamment à la détermination de la saturation résiduelle en huile d'une formation souterraine, en particulier lors de l'exploration et l'exploitation de puits d'hydrocarbures, et particulièrement pour la récupération assistée d'hydrocarbures (EOR : de l'anglais Enhanced Oil Recovery).

On estime aujourd'hui que pour l'ensemble des réservoirs pétroliers actifs, 60%-65% de l'huile en place reste piégée. Différents mécanismes sont à l'origine de ce piégeage, par exemple, l'hétérogénéité géologique engendre un déplacement non homogène de l'huile à l'échelle du réservoir. Mais même à l'échelle locale, la présence d'une tension interfaciale entre l'eau et l'huile conduit à un piégeage capillaire de la phase huile au centre des pores dans le cas des roches mouillables à l'eau. Ce piégeage peut représenter jusqu'à 50% de l'huile en place. Les réservoirs mouillables à l'eau constituent à peu près la moitié des réservoirs dans le monde.

Par conséquent, mobiliser l'huile résiduelle contenue dans la matrice en conditions de mouillabilité préférentielle à l'eau est un véritable défi. Cependant, l'utilisation de tensioactifs injectés en phase aqueuse peut permettre une diminution très importante du piégeage capillaire. Les tensioactifs ont la propriété de pouvoir abaisser très notablement cette tension voire de quasiment l'annuler. L'utilisation d'additifs tensioactifs a été testée avec succès sur les grès dans les années 1980 et connaît un regain d'intérêt. Les procédés de récupération assistée du pétrole (hydrocarbures) par injection de tensioactif représentent un fort potentiel car ils permettent de dépiéger une quantité importante d'huile bloquée dans les pores de la roche.

Pour caractériser le dépiégeage capillaire, il est nécessaire de réaliser des mesures de propriétés relatives de l'écoulement de fluides dans le milieu poreux. Actuellement, les méthodes utilisées nécessitent plusieurs semaines pour obtenir cette caractérisation de l'écoulement de fluides. En effet, il est actuellement nécessaire de préparer des échantillons de roche ayant un volume de pore suffisant pour que les fluides produits et collectés à la sortie du volume poreux aient un volume suffisant pour caractériser les propriétés du milieux (typiquement plusieurs millilitres). Ainsi, les tailles typiques d'échantillon sont de l'ordre de 5 cm de diamètre et 10 cm de longueur. Les échantillons sont ensuite initialement saturés en eau, puis drainés avec de l'huile, et enfin balayés à l'eau pour obtenir la saturation résiduelle en huile. Puis une injection d'un volume d'une formulation ASP (ASP pour Alcalin, surfactant polymère) est réalisée puis une séquence finale de balayage à l'eau. A la fin de la séquence, la saturation résiduelle en huile est déduite du volume totale d'huile produit à la sortie du milieu poreux. WO 2008/132132 A1 décrit un système connu pour l'analyse de l'écoulement d'un fluide dans un milieu poreux par radiographie ou tomographie à rayons X.

Par ailleurs, dans d'autres domaines techniques, il peut être intéressant de caractériser rapidement l'écoulement d'un fluide au sein d'un milieu poreux, notamment un milieu poreux formé par un polymère.

L'invention concerne un système et un procédé de mesure d'au moins une propriété d'écoulement d'au moins un fluide au sein d'un milieu poreux. Le système de mesure comporte au moins une cellule, des moyens d'injection de fluide(s) dans la cellule, et des moyens de radiographie à rayons X. L'utilisation de moyens de radiographie par rayons X dans une cellule, dans laquelle on peut injecter un fluide, permet de réaliser des mesures de manière rapide et en temps réel.

### Le système et le procédé selon l'invention

L'invention concerne un système selon la revendication 1.

En outre, l'invention concerne un procédé de mesure selon la revendication 10.

### Présentation succincte des figures

D'autres caractéristiques et avantages du système et du procédé selon l'invention, apparaîtront à la lecture de la description ci-après d'exemples non limitatifs de réalisations, en se référant aux figures annexées et décrites ci-après.
La figure 1 illustre un système de mesure selon un mode de réalisation de l'invention.
La figure 2 illustre la saturation moyenne en huile et la différence de pression pour un exemple de procédé selon l'invention.
La figure 3 illustre le profil de saturation en huile le long de l'échantillon pendant la phase de drainage à plusieurs instants pour l'exemple de la figure 2.
La figure 4 illustre le profil de saturation en huile le long de l'échantillon pendant la phase de balayage avec des tensioactifs pour plusieurs débits pour l'exemple de la figure 2.
La figure 5 représente la saturation résiduelle mesurée avec le système en fonction du nombre capillaire de balayage pour deux types de roche pour l'exemple de la figure 2.

### Description détaillée de l'invention

La présente invention concerne un système de mesure d'au moins une propriété d'écoulement d'au moins un fluide au sein d'un milieu poreux. Les propriétés d'écoulement sont la saturation moyenne du fluide et/ou le profil de saturation du fluide. Les propriétés de l'écoulement peuvent être dépendants des paramètres opérationnels ainsi que des débits de chaque fluide. Le fluide peut être de tout type, il peut comporter notamment une phase aqueuse, une phase huile... Lorsque le fluide comporte une phase aqueuse, il peut comporter en outre au moins un additif, par exemple un tensioactif. Les tensioactifs ont la propriété de pouvoir abaisser très notablement la tension interfaciale entre l'eau et l'huile, voire de quasiment l'annuler. Le milieu poreux peut être de tout type, il peut être formé notamment par un échantillon d'une roche prélevée dans une formation souterraine, ou être un polymère poreux. Dans le cas où le milieu poreux est une roche, il est possible de caractériser les écoulements de fluide(s) dans la formation souterraine.

Le système de mesure selon l'invention comporte :
- au moins une cellule apte à contenir le milieu poreux, la cellule peut être appelée porte-échantillon, le système de mesure peut comporter plusieurs cellules afin de réaliser des mesures simultanées,
- des moyens d'injection du fluide dans la cellule, les moyens d'injection sont aptes à faire circuler au moins un fluide dans la cellule, par exemple, les moyens d'injection peuvent comprendre au moins un réservoir de fluide, au moins une conduite reliant le réservoir à la cellule, au moins une pompe, au moins une vanne et au moins un capteur de pression,
- des moyens de radiographie à rayons X, les moyens de radiographie à rayons X comprennent au moins une source de rayons X, et un détecteur de rayons X, de préférence un détecteur plan, la cellule étant positionnée entre la source et le détecteur, les moyens de radiographie sont aptes à réaliser une pluralité de radiographies lors de l'injection d'au moins un fluide dans la cellule.

Les moyens de radiographie à rayons X permettent de réaliser des mesures de propriétés d'écoulement du fluide dans le milieu poreux contenu dans la cellule. En effet, par calibration il est possible de relier le niveau de gris de l'image de radiographie obtenue à la saturation dans le milieu poreux. Pour cela, une image préalable peut être réalisée avec l'échantillon saturée d'au moins un fluide à injecter dans le milieu poreux. Ainsi, il est possible d'avoir des radiographies dites « de référence », à comparer avec les radiographies en cours d'injection. Par exemple, dans le cas d'une mesure relative à un échantillon de roche, une première radiographie peut être réalisée pour un échantillon saturé d'eau, puis une deuxième radiographie peut être réalisée pour un échantillon saturé d'huile. L'utilisation d'une pluralité de radiographies permet également une mesure rapide (de l'ordre de grandeur de l'heure à comparer aux quelques semaines nécessaires pour les mesures actuelles) et en temps réel.

Pour des raisons de sécurité, les moyens de radiographie et la cellule peuvent être à l'intérieur d'une cabine de protection des rayons X, alors que les autres composants du système de mesure, notamment les moyens d'injection de fluide peuvent être à l'extérieur de la cabine de protection. Le système comprend encore des moyens de collecte et d'analyse des radiographies obtenues par le détecteur, des moyens de radiographie, les moyens de collecte et d'analyse permettent de stocker et analyser les mesures obtenues par les radiographies, les moyens de collecte et d'analyse comprennent un système informatique, les moyens de collecter et d'analyse sont configuré pour relier le niveau de gris de chaque image de radiographie à la saturation dans le milieu poreux, les moyens de collecte et d'analyse peuvent aussi servir de moyens de visualisation des mesures.

En outre, le système de mesure peut comporter les éléments suivants, seuls ou en combinaison :
- des moyens de commande des moyens d'injection, par exemple les moyens de commande sont aptes à commander au moins une pompe, au moins une vanne et, au moins un débitmètre, etc., les moyens de commande peuvent comprendre un système informatique,
- des moyens de déplacement de la cellule, les moyens de déplacement peuvent être aptes à déplacer la cellule par des translations selon deux directions horizontales et selon une direction verticale, ainsi, il est possible d'adapter le système de mesure à différentes dimensions de la cellule.

Dans le cas où le système de mesure comporte à la fois des moyens de commande des moyens d'injection et des moyens de collecte et/ou d'analyse des radiographies, un unique système informatique peut réaliser ces deux fonctions.

Selon un mode de réalisation de l'invention, la cellule du système de mesure peut avoir une forme sensiblement cylindrique. De préférence, la cellule possède des faibles dimensions (on parle de mini-échantillon) par rapport aux dimensions classiques utilisées pour les mesures de saturation en huile. Ces dimensions classiques sont de l'ordre de 5 cm de diamètre et 10 cm de longueur. Selon une conception possible de la cellule, le diamètre de la cellule (ou le diamètre dans lequel est inscrite la cellule lorsque celle-ci n'est pas de forme cylindrique) est sensiblement compris entre 2 mm et 3 cm, de préférence entre 5 mm et 2 cm. Par exemple, le diamètre de la cellule peut être d'environ 1 cm. La longueur (hauteur) de la cellule est comprise entre 5 et 50 mm, et peut valoir par exemple environ 20 mm. Les dimensions réduites de la cellule par rapport aux échantillons classiquement utilisés permettent de réaliser des mesures de manière plus rapide, notamment car les temps d'injection peuvent être plus courts. Grâce aux temps d'expérience plus courts, les mini-échantillons permettent également de réaliser plusieurs expériences et d'étudier de manière statistique les résultats recherchés.

Selon un mode de réalisation de l'invention, la cellule peut être prévue pour travailler à des températures proches de 150°C, et à des pressions proches de 150 bars.

Les moyens d'injection du fluide dans la cellule peuvent être aptes à injecter un unique fluide. Alternativement, les moyens d'injection peuvent être aptes à injecter une pluralité, par exemple de 2 à 5, fluides dans la cellule. Dans ce cas, l'injection des différents fluides peut être réalisée de manière séquentielle, ou de manière simultanée. La possibilité de pouvoir injecter différents fluides permet de prévoir des séquences d'injection particulières pour déterminer certaines propriétés de l'écoulement dans l'échantillon. Par exemple, dans le cas de l'étude d'un tensioactif pour un procédé EOR, il est envisageable de prévoir trois fluides à injecter : de l'eau, de l'huile et une phase aqueuse comprenant un tensioactif.

De plus, les moyens d'injection du fluide dans la cellule peuvent comporter des moyens pour ajuster le débit d'injection du fluide dans la cellule, par exemple un débitmètre, afin de réaliser des mesures dépendant du débit.

En outre, les moyens d'injection du fluide peuvent comprendre au moins un régulateur de pression, pour régler la pression du fluide injecté dans la cellule.

Les moyens de radiographie à rayons X sont prévus pour réaliser des radiographies à intervalles réguliers pendant la ou les injections de fluide(s). L'intervalle régulier est compris entre 0,1 et 5 secondes, et peut valoir par exemple 1 seconde. Ainsi, la réalisation de radiographies à intervalles réguliers permet de suivre régulièrement les écoulements dans la cellule, ce qui permet un suivi en temps réel des écoulements dans l'échantillon.

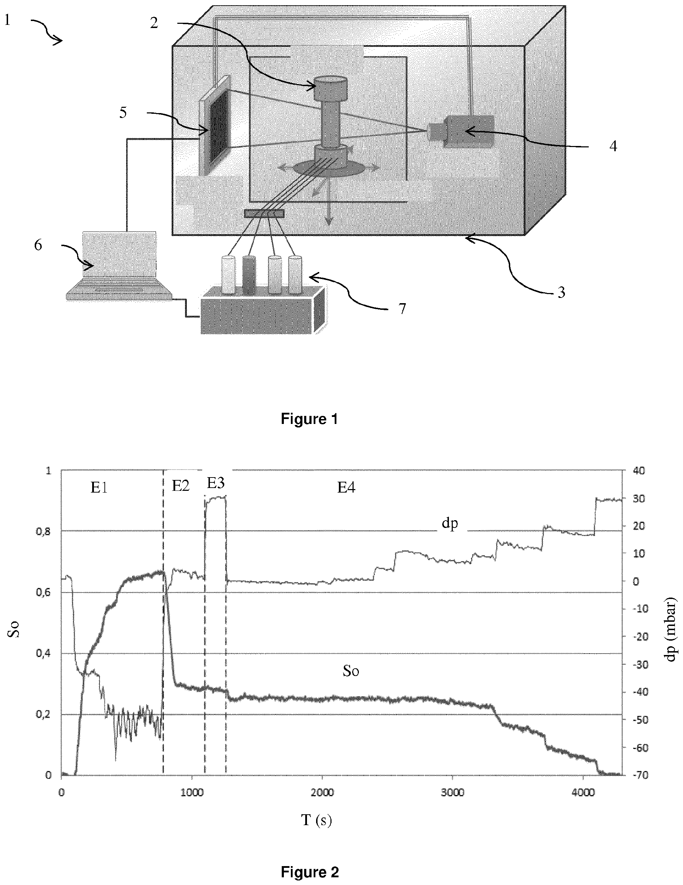

La figure 1 illustre, de manière schématique et non limitative, un système de mesure selon un mode de réalisation de l'invention. Le système de mesure 1 comporte une cellule 2 qui contient un échantillon de roche (non représenté), ou tout autre milieu poreux. La cellule 2 est placée dans une cabine de protection 3 des rayons X. Dans la cabine 3 se trouve également des moyens de radiographie à rayons X qui comportent une source 4 de rayons X et un détecteur 5 de rayons X. La cellule 2 est placée sur un support qui peut être déplacé selon trois axes (représentés schématiquement par des flèches). Le système de mesure 1 comporte également des moyens d'injection 7 de fluide dans la cellule. Les moyens d'injection 7 sont prévus avec quatre fluides. Les moyens d'injection 7 sont reliées à la cellule par quatre conduites. Les moyens d'injection 7 sont disposés en dehors de la cabine de protection 3 des rayons X. En outre, le système de mesure 1 comporte un système informatique 6. Le système informatique 6 est relié au détecteur 5 et aux moyens d'injection 7. Le système informatique 6 sert de moyens de commande des moyens d'injection 7 et de moyens de collecte et d'analyse des radiographies obtenues par le détecteur 5. Le système informatique 6 est disposé en dehors de la cabine de protection 3.

La présente invention concerne également un procédé de mesure d'au moins une propriété d'écoulement d'au moins un fluide au sein d'un milieu poreux. Le procédé de mesure selon l'invention se base sur l'utilisation du système de mesure selon l'invention.

Le procédé selon l'invention comporte les étapes définit dans la revendication 10 et en particulier les étapes suivantes :
- on place un milieu poreux à analyser dans la cellule du système de mesure ;
- on injecte un ou plusieurs fluides dans la cellule par les moyens d'injection de fluide du système de mesure ;
- pendant l'injection on réalise une pluralité de radiographies à rayons X avec les moyens de radiographie du système de mesure ; et
- on détermine au moins une propriété d'écoulement au moyen des radiographies.

Au moyen du procédé selon l'invention, on mesure au moins une des propriétés suivantes : la saturation moyenne d'un fluide injecté au sein du milieu poreux, le profil de saturation d'un fluide injecté au sein du milieu poreux.

En outre, au moyen de ces valeurs, on peut mesurer une saturation résiduelle (par exemple en huile) pour différents débits de balayage (injection) en phase aqueuse comprenant un tensioactif. Il est alors possible de tracer une courbe de saturation résiduelle en fonction du nombre capillaire de balayage. La courbe représentant l'évolution de la saturation résiduelle en huile en fonction du nombre capillaire est appelé CDC (Courbe de Désaturation Capillaire). Elle peut représenter la quantité d'huile productible par un procédé EOR par injection d'une composition aqueuse comprenant au moins un tensioactif. La CDC joue donc un rôle important dans un procédé EOR. Cette courbe dépend notamment de la nature de la roche.

Ainsi, le procédé selon l'invention peut être utilisé dans le cadre d'un procédé de récupération assistée d'hydrocarbures (EOR), pour lequel on utilise le procédé selon l'invention avec le système de mesure selon l'invention, pour déterminer la formulation de la composition (eau et au moins un additif, dont un tensioactif) injectée dans la formation souterraine. Dans ce cas, le milieu poreux utilisé correspond à un échantillon de roche prélevé dans la formation souterraine, dans laquelle le procédé EOR est mis en œuvre.

Conformément à l'invention, les moyens de radiographie à rayons X sont prévus pour réaliser des radiographies à intervalles réguliers pendant la ou les injections de fluide. L'intervalle régulier est compris entre 0,1 et 5 secondes, et peut valoir par exemple 1 seconde. Ainsi, la réalisation de radiographies à intervalles réguliers permet de suivre régulièrement les écoulements dans la cellule, ce qui permet un suivi en temps réel des écoulements dans l'échantillon.

Selon un mode de réalisation, pour lequel on analyse l'intérêt de l'injection d'un tensioactif dans une roche, le procédé selon l'invention peut comprendre la séquence d'injection suivante :
- drainage à l'huile avec plusieurs débits différents, c'est-à-dire injection d'huile pour drainer l'eau présente dans l'échantillon, par exemple à trois débits différents,
- balayage à l'eau à plusieurs débits différents, c'est-à-dire injection d'eau pour éliminer le surplus d'huile, par exemple à deux débits différents, le balayage à l'eau peut se réaliser à débit très faible voire nul, dans ce cas, l'eau balaie le milieu poreux uniquement par les forces capillaires, on parle d'imbibition spontanée.
- injection d'une phase aqueuse comprenant un tensioactif (surfactant) à différents débits croissants, par exemple huit débits différents, jusqu'à désaturation totale de l'huile.

Pendant ces quatre étapes, des radiographies de la cellule sont réalisées.

De plus, cette séquence peut comprendre les étapes suivantes :
- injection d'un volume d'eau de balayage, et
- rinçage et nettoyage de l'échantillon.

Selon une alternative, le procédé selon l'invention peut comprendre l'injection simultanée d'eau et huile pour différents débits. Ces injections communes permettent notamment une mesure de la perméabilité relative.

### Exemple

Le système et le procédé selon l'invention sont mis en œuvre lors d'une séquence ayant pour but de caractériser l'évolution de la saturation résiduelle en huile lors de différents balayages à l'eau et un tensioactif à différent débits.

Un échantillon de grès de diamètre 10 mm et de longueur 19 mm est utilisé dans une des cellules. Au départ, l'échantillon est saturé à 100% en eau. La séquence d'injection réalisée par le système de mesure illustré en figure 1 est la suivante :
- E1 : Drainage à l'huile à trois débits différents,
- E2 : Imbibition spontanée,
- E3 : Balayage à l'eau à deux débits donnés, et
- E4 : Injection de tensioactif en phase aqueuse à huit débits croissants jusqu'à désaturation totale de l'huile.

Au cours de l'expérience, une radiographie est enregistrée chaque seconde permettant après calibration d'obtenir à la fois la saturation moyenne en huile et le profil de saturation le long de l'échantillon. La différence de pression au cours du temps est également mesurée. La saturation moyenne So et la différence de pression dp (mbar) en fonction du temps T (s) sont représentés sur la figure 2 pour la séquence d'injection E1 à E4. L'ensemble du cycle est réalisé en 4000 secondes soit un peu plus d'une heure. On remarque que l'injection de tensioactif en phase aqueuse permet d'extraire la totalité de l'huile présente dans l'échantillon : à la fin de la séquence So=0. Simultanément, la différence de pression augmente dans l'échantillon.

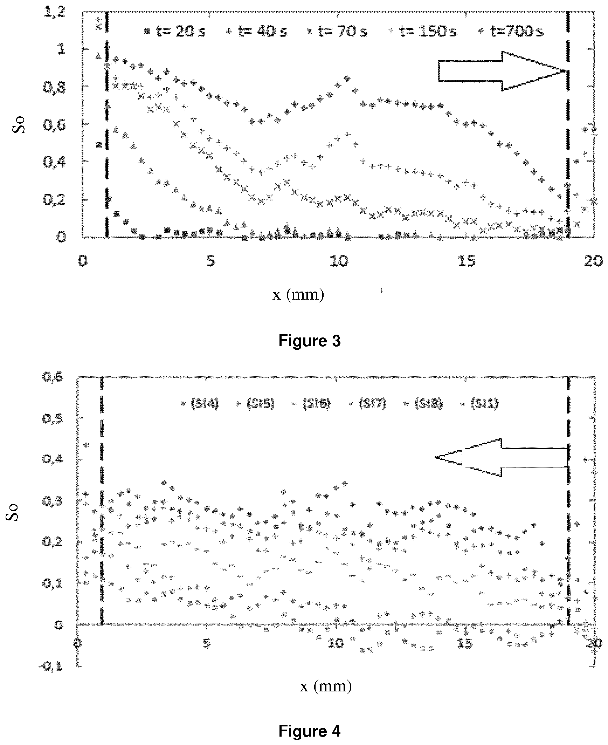

Les profils de saturation pour chaque séquence sont également obtenus à partir de chaque radiographie mesurée. Le traitement réalisé est une moyenne glissante le long de l'image. Ajouté à la calibration, on peut obtenir la saturation en huile le long de l'échantillon chaque seconde. La figure 3 et la figure 4 donnent deux exemples de profils de saturation en huile So en fonction de la profondeur de l'échantillon x (mm) obtenus pendant la phase de drainage E1 et pendant la phase de balayage avec le tensioactif E4. Sur ces figures, les limites des échantillons sont symbolisés par les traits verticaux discontinus, de plus le sens de l'injection est représenté par une flèche. La figure 3 représente l'évolution de la saturation So à différents instants : t=20 s, t=40 s, t= 70 s, t=150 s et t=700 s. Comme attendu, la saturation en huile augmente pendant la phase de drainage. La figure 4 représente l'évolution de la saturation So à la fin de différentes séquences d'injection d'eau et de tensioactifs à différents débits noté Si4, Si5, Si6, Si7, Si8 et Si11 (pour Surfactant Injection : SI), avec SI4 : 0.16 cm³/min, SI5 : 0.25 cm³/min, SI6 : 0.5 cm³/min, SI7 : 1cm³/min, SI8 : 2 cm³/min et SI1 : 0.02 cm³/min.

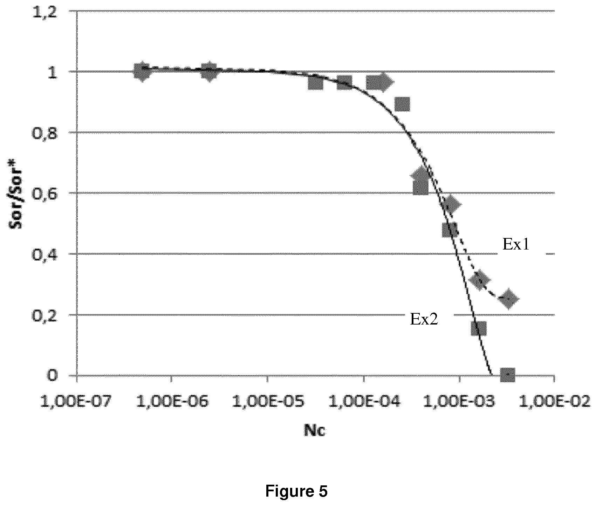

Les profils obtenus permettent de mesurer la saturation résiduelle pour chaque palier de balayage en eau et tensioactifs E4. Il est alors possible de tracer la courbe de saturation résiduelle Sor/Sor* (avec Sor saturation résiduelle et Sor* saturation résiduelle normalisée) en fonction du nombre capillaire Nc de balayage (figure 5) une donnée importante pour toute étude EOR. Le nombre capillaire Nc est le rapport entre la vitesse moyenne de balayage fois la viscosité de l'eau divisé par la tension interfaciale entre le système eau/tensioactif et l'huile. Sur la figure 5, deux courbes sont obtenues pour deux échantillons de grès différents avec la même séquence expérimentale: il s'agit du grès de Bentheimer et du grès de Clashach.

Ainsi, en environ une heure, il est possible d'obtenir une courbe CDC précise, au contraire des méthodes classiques qui peuvent nécessiter plusieurs semaines.

## Revendications

1. Système de mesure de la saturation moyenne et/ou du profil de saturation d'au moins un fluide au sein d'un milieu poreux, ledit système de mesure (1) comporte au moins une cellule (2) contenant ledit milieu poreux, des moyens d'injection (7) dudit fluide dans ladite cellule (2), et des moyens de radiographie à rayons X (3) comprenant une source (4) et un détecteur (5), **caractérisé en ce que** le diamètre de ladite cellule (2) est sensiblement compris entre 2 mm et 5 cm, **en ce que** la longueur de ladite cellule (2) est compris entre 5 et 50 mm, et **en ce que** lesdits moyens de radiographie à rayon X (3) étant aptes à réaliser une pluralité de radiographies lors de l'injection dudit fluide à intervalles réguliers, sensiblement compris entre 0,1 et 5 secondes, pour suivre régulièrement en temps réel les écoulements dans la cellule, et **en ce que** ledit système de mesure comporte des moyens de collecte et d'analyse (6) desdites radiographies obtenues par ledit détecteur (5) permettant de stocker et analyser les mesures obtenues par les radiographies, lesdits moyens de collecte et d'analyse (6) comprenant un système informatique pour relier le niveau de gris de chaque image de radiographie à la saturation du fluide dans le milieu poreux par comparaison des radiographies en cours d'injection avec au moins une radiographie de référence, ladite radiographie de référence correspondant à une image dudit milieu poreux saturé en ledit fluide à injecter dans ledit milieu poreux.

2. Système selon la revendication 1, dans lequel ledit système de mesure comporte des moyens de commande desdits moyens d'injection (6).

3. Système selon l'une des revendications précédentes, dans lequel ladite cellule (2) a une forme sensiblement cylindrique.

4. Système selon la revendication 3, dans lequel le diamètre de ladite cellule (2) est sensiblement compris 5 mm et 2 cm.

5. Système selon l'une des revendications précédentes, dans lequel lesdits moyens d'injection (7) comprennent une pluralité de fluides à injecter dans ladite cellule (2), de manière simultanée ou séquentielle.

6. Système selon l'une des revendications précédentes, dans lequel lesdits moyens d'injection (7) comprennent au moins une pompe, au moins une vanne et au moins un capteur de pression.

7. Système selon l'une des revendications précédentes, dans lequel ledit fluide est choisi parmi une phase aqueuse et une phase huile.

8. Système selon la revendication 7, dans lequel au moins une phase aqueuse comporte au moins un additif, notamment un tensioactif.

9. Système selon l'une des revendications précédentes, dans lequel ledit système de mesure comporte des moyens de positionnement de ladite cellule (2).

10. Procédé de mesure de la saturation moyenne et/ou du profil de saturation d'au moins un fluide au sein d'un milieu poreux, dans lequel on réalise lesdites mesures au moyen dudit système de mesure (1) selon l'une des revendications précédentes, au moyen d'une pluralité de radiographies lors de l'injection dudit fluide à intervalles réguliers, sensiblement compris entre 0,1 et 5 secondes, pour suivre régulièrement les écoulements dans la cellule, dans lequel on collecte et/ou on analyse lesdites radiographies, et on relie le niveau de gris de chaque image de radiographie à la saturation du fluide dans le milieu poreux par comparaison des radiographies en cours d'injection avec au moins une radiographie de référence du milieu poreux saturé d'au moins un fluide, ladite radiographie de référence étant réalisée au préalable en saturant ledit milieu poreux dudit fluide à injecter dans ledit milieu poreux.

11. Procédé selon la revendication 10, dans lequel on trace une courbe de saturation résiduelle en fonction du nombre capillaire de balayage au moyen desdites mesures.

12. Procédé selon l'une des revendications 10 à 11, dans lequel on réalise les mesures par radiographie à rayon X au moyen dudit système de mesure (1) lors de la mise en oeuvre des étapes suivantes d'injection au sein de ladite cellule :
a) on injecte de l'huile à au moins deux débits différents ;
b) on injecte de l'eau à au moins deux débits différents ; et
c) on injecte une phase aqueuse comprenant au moins un additif à au moins deux débits différents.

## Patentansprüche

1. System zur Messung der mittleren Sättigung und/oder des Sättigungsprofils mindestens eines Fluids innerhalb eines porösen Mediums, wobei das Messsystem (1) mindestens eine Zelle (2), die das poröse Medium enthält, Mittel zum Injizieren (7) des Fluids in die Zelle (2) und Röntgenstrahlen-Radiographiemittel (3) mit einer Quelle (4) und einem Detektor (5) aufweist, **dadurch gekennzeichnet, dass** der Durchmesser der Zelle (2) im Wesentlichen zwischen 2 mm und 5 cm beträgt, dadurch, dass die Länge der Zelle (2) zwischen 5 und 50 mm beträgt, und dadurch, dass die Röntgenstrahlen-Radiographiemittel (3) geeignet sind, eine Mehrzahl von Radiographien bei der Injektion des Fluids in regelmäßigen Abständen vorzunehmen, die im Wesentlichen zwischen 0,1 und 5 Sekunden betragen, um die Strömungen in der Zelle regelmäßig in Echtzeit zu verfolgen, und dadurch, dass das Messsystem Mittel zur Sammlung und Analyse (6) der durch den Detektor (5) erhaltenen Radiographien aufweist, die es gestatten, die durch die Radiographien erhaltenen Messungen zu speichern und zu analysieren, wobei die Mittel zur Sammlung und Analyse (6) ein Computersystem umfassen, um den Grauwert jedes Radiographiebilds mit der Sättigung des Fluids im porösen Medium durch Vergleich der Radiographien bei der Injektion mit mindestens einer Referenzradiographie zu verbinden, wobei die Referenzradiographie einem Bild des porösen Mediums entspricht, das mit dem Fluid gesättigt ist, das in das poröse Medium zu injizieren ist.

2. System nach Anspruch 1, wobei das Messsystem Steuermittel der Injektionsmittel (6) aufweist.

3. System nach einem der vorhergehenden Ansprüche, wobei die Zelle (2) eine im Wesentlichen zylindrische Form aufweist.

4. System nach Anspruch 3, wobei der Durchmesser der Zelle (2) im Wesentlichen zwischen 5 mm und 2 cm beträgt.

5. System nach einem der vorhergehenden Ansprüche, wobei die Injektionsmittel (7) eine Mehrzahl von Fluiden umfassen, die gleichzeitig oder sequentiell in die Zelle (2) zu injizieren sind.

6. System nach einem der vorhergehenden Ansprüche, wobei die Injektionsmittel (7) mindestens eine Pumpe, mindestens ein Ventil und mindestens einen Drucksensor umfassen.

7. System nach einem der vorhergehenden Ansprüche, wobei das Fluid aus einer wässrigen Phase und einer Ölphase gewählt ist.

8. System nach Anspruch 7, wobei mindestens eine wässrige Phase mindestens ein Additiv aufweist, insbesondere ein Tensid.

9. System nach einem der vorhergehenden Ansprüche, wobei das Messsystem Mittel zur Positionierung der Zelle (2) aufweist.

10. Verfahren zur Messung der mittleren Sättigung und/oder des Sättigungsprofils mindestens eines Fluids innerhalb eines porösen Mediums, wobei die Messungen mittels des Messsystems (1) nach einem der vorhergehenden Ansprüche mittels einer Mehrzahl von Radiographien bei der Injektion des Fluids in regelmäßigen Abständen vorgenommen werden, die im Wesentlichen zwischen 0,1 und 5 Sekunden betragen, um die Strömungen in der Zelle regelmäßig zu verfolgen, wobei die Radiographien gesammelt und/oder analysiert werden und der Grauwert jedes Radiographiebilds mit der Sättigung des Fluids im porösen Medium durch Vergleich der Radiographien bei der Injektion mit mindestens einer Referenzradiographie des porösen Mediums, das mit mindestens einem Fluid gesättigt ist, verbunden wird, wobei die Referenzradiographie zuvor durch Sättigung des porösen Mediums mit dem in das poröse Medium zu injizierenden Fluid vorgenommen wird.

11. Verfahren nach Anspruch 10, wobei eine Restsättigungskurve in Abhängigkeit von der Spülungs-Kapillarzahl mittels der Messungen erstellt wird.

12. Verfahren nach einem der Ansprüche 10 bis 11, wobei die Messungen durch Röntgenstrahlen-Radiographie mittels des Messsystems (1) bei der Durchführung der folgenden Schritte des Injizierens in die Zelle vorgenommen werden:
a) es wird Öl bei mindestens zwei verschiedenen Duchflussgeschwindigkeiten injiziert;
b) es wird Wasser bei mindestens zwei verschiedenen Duchflussgeschwindigkeiten injiziert; und
c) es wird eine wässrige Phase mit mindestens einem Additiv bei mindestens zwei verschiedenen Duchflussgeschwindigkeiten injiziert.

## Claims

1. System for measuring the average saturation and/or the saturation profile of at least one fluid within a porous medium, said measurement system (1) having at least one cell (2) containing said porous medium, means (7) for injecting said fluid into said cell (2), and X-ray radiography means (3) comprising a source (4) and a detector (5), **characterized in that** the diameter of said cell (2) is substantially between 2 mm and 5 cm, **in that** the length of said cell (2) is between 5 and 50 mm, and **in that** said X-ray radiography means (3) are able to take a plurality of X-rays during the injection of said fluid at regular intervals, substantially between 0.1 and 5 seconds, so as to regularly monitor the flows in the cell in real time, and **in that** said measurement system has means (6) for collecting and analysing said X-rays obtained by said detector (5) that make it possible to store and analyse the measurements obtained by the X-rays, said collection and analysis means (6) comprising a computer system for linking the greyscale level of each X-ray image to the saturation of the fluid in the porous medium by comparing the X-rays during injection with at least one reference X-ray, said reference X-ray corresponding to an image of said porous medium saturated with said fluid to be injected into said porous medium.

2. System according to Claim 1, wherein said measurement system has means for controlling said injection means (6).

3. System according to either of the preceding claims, wherein said cell (2) has a substantially cylindrical shape.

4. System according to Claim 3, wherein the diameter of said cell (2) is substantially between 5 mm and 2 cm.

5. System according to one of the preceding claims, wherein said injection means (7) comprise a plurality of fluids to be injected into said cell (2), simultaneously or sequentially.

6. System according to one of the preceding claims, wherein said injection means (7) comprise at least one pump, at least one valve and at least one pressure sensor.

7. System according to one of the preceding claims, wherein said fluid is chosen from an aqueous phase and an oil phase.

8. System according to Claim 7, wherein at least one aqueous phase has at least one additive, in particular a surfactant.

9. System according to one of the preceding claims, wherein said measurement system has means for positioning said cell (2).

10. Method for measuring the average saturation and/or the saturation profile of at least one fluid inside a porous medium, wherein said measurements are taken by means of said measurement system (1) according to one of the preceding claims, by means of a plurality of X-rays during the injection of said fluid at regular intervals, substantially between 0.1 and 5 seconds, so as to regularly monitor the flows in the cell, wherein said X-rays are collected and/or analysed, and the greyscale level of each X-ray image is linked to the saturation of the fluid in the porous medium by comparing the X-rays during injection with at least one reference X-ray of the porous medium saturated with at least one fluid, said reference X-ray being taken beforehand by saturating said porous medium with said fluid to be injected into said porous medium.

11. Method according to Claim 10, wherein a curve of residual saturation as a function of the flushing capillary number is plotted by means of said measurements.

12. Method according to either of Claims 10 and 11, wherein the X-ray radiography measurements are taken by means of said measurement system (1) during the implementation of the following steps of injection into said cell:
a) oil is injected at at least two different flow rates;
b) water is injected at at least two different flow rates; and
c) an aqueous phase comprising at least one additive is injected at at least two different flow rates.
